# EUROPEAN PATENT APPLICATION

(11) **EP 2 269 565 A1**
(43) Date of publication of application: **05.01.2011**
(21) Application number: 09163865.0
(22) Date of filing: 26.06.2009
(51) Int. Cl.: A61K 8/06, A61K 8/25, A61K 8/26, A61K 8/28, A61Q 15/00

(54) **Antiperspirant compositions containing a fragrance oil**

(71) Applicant: Unilever PLC, 100 Victoria Embankment London, Greater London EC4Y 0DY (GB); UNILEVER NV, 3013 AL Rotterdam (NL)
(72) Inventor: Cordero Martin, Vanessa, Leeds, LS14 2AR Yorkshire (GB); Pedley, Christopher Neil, Leeds, LS14 2AR Yorkshire (GB)
(74) Representative: Whaley, Christopher

(57) **Abstract**

An antiperspirant composition in the form of oil in water emulsions containing a comparatively high concentration of both an aluminium-zirconium astringent salt and fragrance oil suffers from instability during storage prior to use. Such compositions have a Fragrance Index of at least 15. Instability is ameliorated by incorporating a hydrophobically treated particulate silica, especially fumed silica to generate a Stability Index in the range of from 4 to 15.

## Description

The present invention relates to antiperspirant compositions containing a fragrance oil, particularly to such compositions in the form of oil in water compositions and more particularly to compositions containing a high concentration of an antiperspirant salt.

### Background and Prior Art

Humans sweat through eccrine glands as a mechanism to control body temperature. The distribution of eccrine glands in the skin is uneven and particularly concentrated in areas such as the armpits (axillae). As a consequence, in such localised areas wet patches become noticeable, and can be transferred there to clothing coming into intimate contact with the skin. An industry has developed over the last 100 years to inhibit localised perspiration, and in particular, active materials have been developed that block the eccrine gland ducts, including aluminium zirconium chlorohydrate, optionally complexed. Moreover, various types of compositions have been developed for carrying the antiperspirant active and dispensers for such compositions.

One commonly employed type of dispenser is often called a roll-on, in which a roller, most usually a spherical ball, partly protrudes outside a housing at the mouth of the dispenser that enables the roller to rotate and transport fluid from within the dispenser by adherence to its surface. One common class of formulation suitable for use in a roll-on comprises an oil phase dispersed within an aqueous phase, which in this specification will be called an oil-in-water emulsion. Users apply the composition by rolling the roller across the skin surface. Broadly speaking, each user adopts his or her application habit, applying a similar volume of composition to the skin. As a consequence, the efficacy of an antiperspirant composition increases in accordance with an increase in concentration of the antiperspirant active in the composition, though not necessarily linearly. Moreover, in general, and in a head to head comparison at the same concentration, antiperspirant chlorohydrate actives combining both aluminium and zirconium are more efficacious than those based solely on aluminium. Accordingly, in order to achieve high efficacy, it is desirable not only to select aluminium/zirconium actives, but also to employ them at a high concentration.

Many consumers like to smell an antiperspirant composition at the time of its application to skin. Its fragrance either masks any malodour that has already developed by that time or offers reassurance that malodour that will be generated in the future would be masked. The extent to which any particular fragrance can be detected depends on its concentration in the composition, the higher the concentration, the greater the ability of a human to detect it and to mask malodours and as a first approximation, the longer the fragrance will continue to be detected. Consequently, it is inherently beneficial to be able to incorporate a fragrance at a comparatively high concentration into an antiperspirant composition.

It is inherently desirable that a composition remains stable during storage and transportation prior to use by a consumer. Stable in the context of the present invention refers to physical stability of the emulsion. An unstable emulsion manifests itself by physical separation of aqueous and oil phases. A composition comprising separated phases is likely to deliver a variable ratio of oil to aqueous phase on application, thereby providing a variable sensory perception and even variable performance during the consumption of the product. Such variability is inherently undesirable. For example, the oil phase typically has a lower density than the aqueous phase, which can result in the consumer applying a reduced proportion of fragrance oil from an invert dispenser when the dispenser is full leading to an increased proportion of oil when the dispenser is nearly empty, or if the consumer shakes the dispenser before use, uncontrolled remixing occurs so that the proportion of applied oil phase is not controlled.

Unfortunately, it has been found that although emulsions containing a comparatively low concentration of an aluminium or an aluminium/zirconium antiperspirant active together with a comparatively low concentration of a fragrance are stable, and emulsions containing a high concentration of an aluminium antiperspirant active together with a high concentration of a fragrance are stable, the final combination of emulsions containing a comparatively high concentration of aluminium/zirconium antiperspirant active together with a high concentration of a fragrance are not stable or less stable, especially if they are subjected to elevated temperatures during storage or transportation. Antiperspirant compositions are typically in transit, in warehouses, on stores' shelves or in consumers' homes for many weeks or even months before and during use, so that stability of the product is of significance. Warehouses in particular can reach high temperatures, particularly during summer months and/or in Mediterranean and tropical or semi-tropical climates. Roll-on dispensers are very popular in countries such as Brazil and Mexico where high temperatures often arise. Thus, the producer needs a means to provide an oil-in-water emulsion that combines the high efficacy arising from use of the superior aluminium/zirconium antiperspirant at high concentration with the superior delivery of fragrance arising from a high concentration of fragrance therein. Moreover, the emulsion must retain its ability to be applied from a roll-on dispenser and accordingly must retain a suitably intermediate viscosity, which is to say a viscosity intermediate between that of a cream that is too thick and water that is too thin.

The industry has hitherto contemplated encapsulating the fragrance oil. Although by suitable selection of encapsulating material, namely material that is water-insoluble, but still capable of being ruptured or otherwise releasing the fragrance on topical application, it is possible, at least in theory, to circumvent the problem of separation of the oil phase (which contains the fragrance oil) from the aqueous phase, encapsulation is expensive so that such a procedure inevitably adds significant cost to the product. Hence, an alternative solution remains inherently desirable.

US5618522, Kaleta et al assigned to Procter & Gamble discloses oil in water emulsion compositions in which the oil phase is thickened by particulate materials including polymeric organic particulates, and inorganic clays, silica and calcium silicate, in order to combat greasiness. The text does not disclose incorporating any antiperspirant active, so does not contemplate even the existence of a problem of stability when seeking to employ a high concentration of a selected class of antiperspirant active together with a high concentration of fragrance, let alone instruct the reader on how to solve that problem. Indeed, it is not even suitable for antiperspirant emulsions which contain only a small oil phase, because of the risk of producing a sticky mass of oil and silica rather than a solution.

WO2006/007989, in the name of Unilever, discloses antiperspirant oil in water emulsions containing up to 30% by weight antiperspirant active without discriminating between aluminium actives and aluminium/zirconium actives and also contemplates including a fragrance, but does not recognise the existence of a problem of impaired stability when seeking to employ a high concentration of a selected class of antiperspirant active together with a high concentration of fragrance, let alone instruct the reader on how to solve that problem, even though it does employ a particulate hydrophobically-treated silica in order to accelerate the drying of the emulsion after application to skin.

### Object of the present invention

It is an object of the present invention to improve the stability of oil in water emulsions containing a high concentration of an aluminium zirconium antiperspirant active and a comparatively high concentration of fragrance.

Other and further objects of the present invention may become apparent in the detailed description of the invention hereinafter.

### Brief summary of the present invention

According to one aspect of the present invention, there is provided an oil-in-water emulsion comprising:-
a continuous aqueous phase constituting at least 85% by weight of the emulsion and comprising a solution of an astringent antiperspirant salt,
a disperse oil phase constituting not greater than 10% by weight of the emulsion and comprising at least one water-immiscible oil, including fragrance oil
at least one aliphatic emulsifier at a concentration of at least 1.5% by weight and
a hydrophobic silica
   In which
the antiperspirant salt is an aluminium-zirconium chlorohydrate optionally complexed with glycine at a concentration of at least 16%
the oil phase contains at least 0.7% by weight of fragrance oil
the emulsion satisfies a fragrance index of at least 15 and
the hydrophobic silica is present in an amount that generates a Stability Index of from 4 to 15.

According to a second aspect of the present invention there is provided a method for stabilising an oil-in-water emulsion comprising
a continuous aqueous phase constituting at least 85% by weight of the emulsion and comprising a solution of an astringent antiperspirant salt,
a disperse oil phase constituting not greater than 10% by weight of the emulsion and comprising at least one water-immiscible oil, including fragrance oil
at least one aliphatic emulsifier at a concentration of at least 1.5% by weight and
a hydrophobic silica
   In which emulsion
the antiperspirant salt is an aluminium-zirconium chlorohydrate optionally complexed with glycine and present at a concentration of at least 16%
the oil phase contains at least 0.7% by weight of fragrance oil and
the emulsion satisfies a fragrance index of at least 15 %s being by weight based on the emulsion
by incorporating into the aqueous phase of the emulsion the hydrophobic silica in an amount that generates a Stability Index of from 4 to 15.

By incorporating the hydrophobic silica into the aqueous emulsion containing a comparatively high concentration of each of the selected antiperspirant active salt and the fragrance oil, which emulsion satisfies the Fragrance Index and Stability Index, it is possible to improve the stability of the emulsion.

Herein, the concentration of an ingredient is by weight based on the weight of the emulsion, unless, and except where expressly indicated.

Herein, the term "oil" signifies a liquid organic material at 20°C that is water-insoluble and excludes a material that is water-soluble, even if it is oil-miscible. Any material having a solubility of less than 0.1g/100g at 20°C is considered to be insoluble.

Herein, the value of the Fragrance Index is obtained by multiplying the concentration of the antiperspirant salt expressed as a weight % by the concentration of the fragrance oil expressed as a weight %. For example, if the concentration of the antiperspirant salt is 21 % and the concentration of the fragrance oil is 1.2%, the Fragrance Index is 25.2.

Herein the value of the Stability Index is obtained by first multiplying the concentration of the hydrophobic silica expressed as a weight % by 10 and then dividing the result by the concentration of the fragrance oil expressed as a weight %. For example if the concentration of the hydrophobic silica is 0.75% and the concentration of the fragrance oil is 1.2%, the Stability Index is 6.25.

### More detailed description of the invention including preferred embodiments

The present invention seeks to ameliorate instability of aqueous emulsions in which the aqueous phase contains a high concentration of an aluminium/zirconium chlorohydrate antiperspirant together with a high concentration of a fragrance oil, instability that is not apparent when a lower concentration of either is employed, or even when a high concentration of an aluminium chlorohydrate (free from zirconium) is employed as the antiperspirant active.

The instant invention contains an aqueous phase in which the aluminium zirconium chlorohydrate, optionally complexed, is dissolved. The mole ratio of (Al + Zr metal) : Cl is often selected in the range of from 0.9:1 :to 1.5:1 and in many instances from 1:1.1 to 1.4:1. The chlorohydrate salt, can, if desired, be complexed with a compatible molecule, commonly containing hydroxyl or carboxylic/amino groups. An especially suitable complexant is an aminoacid. Examples of suitable amino acids include dl-tryptophan, dl-β-phenylalanine, dl-valine, dl-methionine and β-alanine, and preferably glycine which has the formula CH₂(NH₂)COOH.. It is preferable to employ an aluminium-zirconium chlorohydrate that is not complexed.

The concentration of the antiperspirant active is often at least 20% by weight and is usually not higher than 25% by weight of the composition. In many instances, its concentration is at least 21 % by weight of the composition. In some, very suitable compositions its concentration is from 24 to 27% by weight of the aqueous phase, i.e. excluding the weight of the dispersed oil phase, the emulsifier(s) and any dispersed particulates.

A second essential constituent of the emulsion to be stabilised is at least 0.7% by weight of fragrance oil, sometimes alternatively called a perfume oil. Herein, "fragrance" signifies any substance intended to impart an odour to the antiperspirant emulsion. In practice, the fragrance is selected to be attractive to a user. The fragrance oil herein may comprise a single fragrance ingredient or more commonly a plurality of fragrance ingredients, each of which ingredients may be a single natural or synthetic fragrance material or a mixture of fragrance materials. A blend of ingredients usually comprises at least 5 or 10 ingredients, often at least 20 and many include at least 50 ingredients. They impart a distinctive aroma, given descriptive titles such as woody, or citrus or fresh. Such ingredients are well known to the producer of fragrance oils. Fragrance oils are commercially available from suppliers including International Flavours and Fragrances, Givaudan, Quest International, and Symrise. A much longer list is given in "The Art of Fragrance Ingredients ", having web address *http:*/*lwww.perfumerbook.com*/*Fragrance%20Houses.htm*. A listing of fragrance ingredients (including diluents) is given in the International Cosmetic Ingredient Dictionary and Handbook, Eighth Edition, 2nd volume pages 1747 to 1750. A further list of fragrance ingredients is given in a "Compilation of Odor and Taste Threshold Values Data, edited by F A Fazzalari, DFS48A published in 1979 by the American Society for Testing and Materials. Said lists can be consulted by a person wishing to obtain a fragrance oil or a blend of such oils, or to create his own blend, typically by mixing at ambient temperature.

The choice of fragrance ingredients is at the discretion of the emulsion producer, taking into account their published characteristics (which for example may be in book compilations or in data sheets from suppliers or otherwise obtainable by a search for materials on the World Wide Web). The selection will often take into account one or more of such factors as the chemical nature of the ingredient, its boiling point and its odour detection threshold. Oil blends include Bergamot, cedar atlas, cedar wood, clove, geranium, guaiacwood, jasmine, lavender, lemongrass, lily of the valley, lime, neroli, musk, orange blossom, patchouli, peach blossom, petotgrain, pimento, rose, rosemary, and thyme, which can comprise extracts from natural materials and/or their synthetic analogues. The weight proportion of fragrance oil in the emulsion is commonly up to 3% advantageously is at least 1 % and particularly at least 1.1 %. In some very desirable compositions, the fragrance comprises up to 2% w/w of the emulsion and in many compositions up to 1.5% by weight.

The concentrations of the emulsion ingredients herein are selected in combination to generate a Fragrance Index of at least 15. Commonly, the Fragrance Index is up to 60 and in many suitable embodiments is up to 50. In a number of preferred embodiments the Fragrance Index is from 20 to 40. In certain highly desirable compositions, the Fragrance Index is from 22 to 33, and particularly when the emulsion contains from 20 to 24% by weight of the aluminium/zirconium antiperspirant salt.

In addition, the emulsion comprises an emulsification system commensurate with producing an oil-in-water emulsion. Such an emulsifier system conveniently has a mean HLB value in the region of from about 5 to about 12 and particularly from 6 to about 10. An especially desired mean HLB value is from 7 to 9. Such a mean HLB value can be provided by selecting an emulsifier having such an HLB value, or more preferably by employing a combination of at least two emulsifiers, a first (lower) HLB emulsifier having an HLB value in the range of from 2 to 6.5, such as in particular from 4 to 6 and a second (higher) HLB emulsifier having an HLB value in the range of from about 6.5 to 18 and especially from about 12 to about 18. When a combination of emulsifiers is employed, the average HLB value can be calculated as a weight average of the HLB values of the constituent emulsifiers.

An especially desirable range of emulsifiers comprises a hydrophilic moiety provided by a polyalkylene oxide (polyglycol), and a hydrophobic moiety provided by an aliphatic hydrocarbon, preferably containing at least 10 carbons and commonly linear. The hydrophobic and hydrophilic moieties can be linked via an ester or ether linkage, possibly via an intermediate polyol such as glycerol. A preferred range of emulsifiers comprises polyethylene glycol ethers.

Preferably the hydrophobic aliphatic substituent contains at least 12 carbons, and is derivable from lauryl, palmityl, cetyl, stearyl, olearyl and behenyl alcohol, and especially cetyl, stearyl or a mixture of cetyl and stearyl alcohols or from the corresponding carboxylic acids.

The polyalkylene oxide is often selected from polyethylene oxide and polypropylene oxide or a copolymer of ethylene oxide and especially comprises a polyethylene oxide. The number of alkylene oxide and especially of ethoxylate units within suitable emulsifiers is often selected within the range of from 2 to 100. Emulsifiers with a mean number of ethoxylate units in the region of 2 can provide a lower HLB value of below 6.5 and those having at least 4 such units provide a higher HLB value of above 6.5 and especially those containing at least 10 ethoxylate units which provide an HLB value of above 10. A preferred combination comprises a mixture of an ethoxylate containing 2 units and one containing from 10 to 40 units, such as from 15 to 30 or desirably from 20 to 25. Particularly conveniently, the combination of emulsifiers comprises steareth-2 and a selection from steareth-15 to steareth-30.

It is desirable to employ a mixture of ethoxylated alcohol emulsifiers in a weight ratio of emulsifier having a lower HLB value of <6.5 to emulsifier having a higher HLB value of >8 of from 1.5:1 to 6:1 and particularly from 2:1 to 5:1.

The total proportion of emulsifiers in the composition is usually at least 1.5% and particularly at least 2% by weight. Commonly, the emulsifiers are not present at above 6%, often not more than 5% by weight and in many preferred embodiments up to 4% by weight. An especially desirable concentration range for the emulsifiers is from 2.5 to 4% by weight.

In order to ameliorate the emulsion instability in the presence simultaneously of a comparatively high concentration of antiperspirant salt and a high concentration of fragrance, a particulate silica such as an amorphous silica, and especially a fumed silica, is incorporated. It is particularly desirable to employ such a fumed (sometimes called pyrogenic) silica which has been hydrophobically treated. Such materials are commercially available under the name hydrophobic silica. Hydrophobic silicas are obtained by chemically bonding a hydrophobic substituent such as especially a siloxane group onto the surface of the silica, possibly following an intermediate treatment in which the surface of the silica has been rendered hydrophilic. Suitable reactants to generate a hydrophobic substituent include halosilanes and in particular chlorosilanes and methylated silazanes such as hexamethyldisilazane.

Desirably, the silica, such as the fumed silica, and especially the hydrophobic silica has a BET specific surface area of at least 100 m²/g and particularly from 150 to 400 m²/g. The silica comprises very fine particles, fumed silica commonly having a diameter for individual particles of below 40 nm and in many instances at least 99% by weight of below 40 nm. In fumed silica as supplied, some aggregation can occur so that in many embodiments, the supplied silica has an average particle size (diameter) of less than or equal to 1000 nm, preferably less than or equal to 500 nm, i.e. the diameter of the silica particle of average weight. In at least some desirable embodiments, at least 99% by weight of the silica particles, as supplied, are in the range of 10 to 500 nm.

The silica is incorporated in such an amount as to generate a Stability Index of at least 4 and preferably at least 5. Commonly the Stability Index is not greater than 15 and in many suitable emulsions is up to 10. In a number of preferred embodiments, the Stability Index is up to 8. By selecting an Index value within such ranges and particularly with preferred or advantageous ranges, it is possible to achieve an advantageous benefit of enhancing emulsion stability of compositions containing relatively high concentrations of particularly efficacious antiperspirant actives and fragrance oils without impairing the sensory properties of the composition unduly. The silica is normally selected in the range of up to 2% and in many desirable embodiments at up to 1%. A range of from 0.6 to 0.8% by weight is of practical value for the silica. The silica is highly conveniently incorporated into the aqueous phase. It can suitably be incorporated after or preferably before emulsification. As an alternative it can be introduced simultaneously with but separately from the oil phase or a component of the oil phase.

The oil phase can include advantageously one or more emollient oils, for example in a proportion of from 0.5 to 10% w/w of the emulsion particularly at least 1% w/w and especially at least 2% w/w. Often the proportion is up to 6% w/w.

Such oils can suitably be selected from alkyl ether oils having a boiling point of above 100°C and especially above 150°C, including polyalklyeneglycol alkyl ethers. Such ethers desirably comprise between 10 and 20 ethylene glycol or propylene glycol units and the alkyl group commonly contains from 4 to 20 carbon atoms. The preferred ether oils include polypropylene glycol alkyl ethers such as PPG-14-butylether and PPG-15-stearyl ether.

The oils can include one or more triglyceride oils. The triglyceride oils commonly comprise the alkyl residues of aliphatic C₇ to C₂₀ alcohols, the total number of carbon atoms being selected in conjunction with the extent of olefinic unsaturation and/or branching to enable the triglyceride to be liquid at 20°C. One example is jojoba oil. Particularly preferably, in the triglyceride oil the alkyl residues are linear C₁₈ groups having one, two or three olefinic degrees of unsaturation, two or three being optionally conjugated, many of which are extractable from plants (or their synthetic analogues), including triglycerides of oleic acid, linoleic acid, linolenic acid, petroselenic acid, ricinoleic acid, linolenelaidic acid, trans 7-octadecenoic acid, parinaric acid, pinolenic acid, punicic acid, petroselenic acid and stearidonic acid.

Suitable examples of natural plant oils derived from unsaturated C₁₈ acids include coriander seed oil, impatiens balsimina seed oil, parinarium laurinarium kernel fat oil, sabastiana brasilinensis seed oil, dehydrated castor seed oil, borage seed oil, evening primrose oil, aquilegia vulgaris oil, sunflower (seed) oil and safflower oil. Other suitable oils are obtainable from hemp, and maize corn oil. An especially convenient natural oil by virtue of its characteristics and availability comprises sunflower (seed) oil, ranging from those rich in oleic acid glycerides to those rich in linoleic acid glycerides, rich indicating that its content is higher than that of the other named acid.

A further class of emollient oils comprise alkyl or alkyl-aryl ester oils having a boiling point of above 150°C (and a melting point of below 20°C).

Such ester oils include oils containing one or two alkyl groups of 12 to 24 carbon atoms length, including isopropyl myristate, isopropyl palmitate and myristyl palmitate. Other non-volatile ester oils include alkyl or aryl benzoates such C₁₂-₁₅ alkyl benzoate, for example Finsolv TN™ or Finsolv Sun™.

A further class of non-volatile emollient oils comprises non-volatile dimethicones, often comprising phenyl or diphenylene substitution, for example Dow Corning 200 350cps or Dow Corning 556.

The aqueous phase can comprise, if desired, up to 8% w/w, such as at least 1% w/w of a water-soluble di or trihydric humectant, including particularly glycerol or a low molecular weight polyethylene glycol, especially of average molecular weight 200 to 600 daltons,.

The emulsion can include, if desired, from 0.05 to 1% w/w, e.g. 0.1 to 0.2% w/w, of an aminocarboxylic acid chelate or a water-soluble salt thereof, such as ethylenediaminetetra-carboxylic acid or pentetic acid, or a sodium salt of either

The emulsion preferably contains up to 0.2% w/w such as 0.05 to 0.1 % w/w of a preservative such as butylated hydroxytoluene.

A process for making an antiperspirant or deodorant oil-in-water emulsions of the present invention comprises the step of mixing together the ingredients comprising water, an antiperspirant or deodorant active, an oil-in-water emulsifier or mixture of emulsifiers, and the fragrance oil (and any other oil) and shearing the mixture to disperse the oil as droplets within the water. The steps of mixing and shearing can be carried out using the relevant conventional equipment.

Preferably, the emulsion is made by first preparing separate aqueous and oil mixtures which are brought together before shearing. The aqueous phase commonly contains the antiperspirant active. Where a mixed emulsifier system is employed, it is desirable to incorporate any emulsifier having a low HLB value, particularly of <6.5 into the oil phase and an emulsifier having a high HLB value, particularly of >6.5 into the aqueous phase. The temperature of either or both of the respective phases can be raised, where desired, to accelerate dissolution of the emulsifier, for example to above 50°C.

It is highly desirable to incorporate the hydrophobic silica with the aqueous phase, for example by dispersing the silica within the aqueous phase prior to introduction of the oil, or possibly separately and simultaneously with the aqueous phase. It is preferable to incorporate the fragrance oil last of all, particularly when the composition has a temperature of below 50°C and shortly before the entire mixture is sheared, especially when either or both phases have been heated so as to accelerate emulsifier dissolution.

Although the emulsion is subjected to shear to create the dispersed phase, it is preferable to avoid very high intensity shearing. Conventional high shear equipment for making emulsions can be employed.

The invention formulations are very suitable for dispensing via a roll-on dispenser, for example any upright dispenser such as described in EP1175165 or an invert dispenser such as described in USP6511243 or WO2005/007377. Invert indicates that the dispenser stands stably with its dispensing ball below the formulations reservoir.

The invention formulation is applied by rolling the ball of the dispenser topically across the skin surface, depositing a film of fluid on the skin. Commonly the dispenser is wiped across the skin between 4 and 10 strokes, depending on the habit of the user and the diameter of the ball. Commonly from 0.2 to 0.5g fluid is deposited in each armpit per application.

Having given a brief summary of the present invention and preferred embodiments, a more detailed description of certain embodiments will be given by way of example only.

### Examples 1 to 12 and Comparisons A to B

The emulsions described in these Examples and Comparisons were made by the following general method. An aqueous solution of the antiperspirant active and any additional water were charged into a preparation vessel equipped with a stirrer, heater and side-pod and heated to approximately 45°C. The contents were mixed at a low agitation speed of about 1.8 m/s and a solution of high HLB emulsifier in water at 75°C was charged from the side-pod into the preparation vessel. When employed, i.e. in the Examples, the hydrophobic fumed silica was charged into the preparation vessel under vacuum and stirrer speed was increased to about 3.5 m/s to disperse it uniformly. The vessel was isolated and if necessary de-aerated.

The oils and low HLB emulsifier were heated to about 65 to 75°C and charged into the vessel at a flow rate of about 33kg/minute through a recycle loop employing a Silverson™ mixer at about 3000rpm. The vessel stirrer speed was increased to 4.5m/s and the side-pot-washout charged. The last ingredients, namely the fragrance oils, were charged at 40-42°C into the vessel with agitation for 2 minutes at a very slow stirrer speed of about 0.6 m/s. Finally the emulsion was discharged through a shear mixer operating at about 1350rpm to a storage container. Subsequently, emulsion samples were charged into clear glass capped flat sided bottles of 100 mls capacity for testing the physical stability of the formulations under controlled temperature conditions.

**Table 1**

| | |
|---|---|
| ZAG | Aluminium Zirconium Tetrachlorohydrex Gly (46% w/w aqueous solution) |
| ZACH | Aluminium Zirconium pentachlorohydrate (40% w/w aqueous solution) |
| PSE | PPG-15 Stearyl Ether |
| SSO | SUNFLOWER seed Oil |
| E-2 | Steareth-2 (low HLB emulsifier) |
| E-20 | Steareth-20 (high HLB emulsifier) |
| F1 | First Fragrance |
| F2 | Second Fragrance |
| BHT | Butylated Hydroxytoluene |
| H30 | Hydrophobic fumed silica (HDK H30™ [Wacker Chemie] |
| Che | Ethylenediaminetetraacetic acid, disodium salt |
| Aq | Demineralised water |

The formulations of Examples 1 to 8 and Comparison A compared the stability of emulsions containing varying concentrations of aluminium zirconium tetrachlorohydrex Gly and concentrations of different fragrances, and the effect of incorporating the hydrophobic silica. The compositions are summarised in Table 2 below, together with their stability as measured at 43°C.

**Table 2**

| | Ex1 | Ex2 | Ex3 | Ex4 | Ex5 | Ex6 | Ex7 | Ex8 | CompA |
|---|---|---|---|---|---|---|---|---|---|
| | % w/w | | | | | | | | |
| ZAG | 43.5 | 43.5 | 50.0 | 50.0 | 43.5 | 43.5 | 50.0 | 50.0 | 43.5 |
| PSE | 0.00 | 0.00 | 0.00 | 0.00 | 2.00 | 2.00 | 2.00 | 2.00 | |
| SSO | 2.00 | 2.00 | 2.00 | 2.00 | 0.00 | 0.00 | 0.00 | 0.00 | 2.0 |
| E-2 | 2.30 | 2.30 | 2.30 | 2.30 | 2.30 | 2.30 | 2.30 | 2.30 | 2.3 |
| E-20 | 0.90 | 0.90 | 0.90 | 0.90 | 0.90 | 0.90 | 0.90 | 0.90 | 0.9 |
| F1 | 1.2 | 0 | 1.0 | 0 | 1.0 | 0 | 1.2 | 0 | 1.2 |
| F2 | 0 | 1.0 | 0 | 1.2 | 0 | 1.2 | 0 | 1.0 | 0 |
| BHT | 0.05 | 0.05 | 0.05 | 0.05 | 0.05 | 0.05 | 0.05 | 0.05 | 0.05 |
| H30 | 0.70 | 0.70 | 0.70 | 0.70 | 0.70 | 0.70 | 0.70 | 0.70 | 0 |
| Che | 0.10 | 0.10 | 0.10 | 0.10 | 0.10 | 0.10 | 0.10 | 0.10 | 0.1 |
| Aq | Balance | | | | | | | | |

| Stability at 43°C (days) | | | | | | | | | |
|---|---|---|---|---|---|---|---|---|---|
| | 128 | 67 | 80 | 29 | 158 | 74 | 53 | 53 | 7 |

Table 2 demonstrates very clearly that the incorporation of the hydrophobic silica has an extremely beneficial effect on stability, the stability increasing from only 7 to 128 days in the test (example 1 versus Comparison A.)

Formulations containing high concentrations of aluminium zirconium pentachorohydrate and fragrance oils are summarised in Table 3 below.

**Table 3**

| | **Ex9** | **Ex10** | **Ex11** | **Ex12** | **CompB** |
|---|---|---|---|---|---|
| | % by weight | | | | |
| **ZACH** | 50.00 | 57.50 | 50.00 | 57.50 | 57.50 |
| **SSO** | 2.00 | 2.00 | 2.00 | 2.00 | 2.00 |
| **E2** | 2.30 | 2.30 | 2.30 | 2.30 | 2.30 |
| **E20** | 0.90 | 0.90 | 0.90 | 0.90 | 0.90 |
| **F1** | 1.0 | 1.2 | 0 | 0 | 0 |
| **F2** | 0 | 0 | 1.0 | 1.2 | 1.2 |
| **BHT** | 0.05 | 0.05 | 0.05 | 0.05 | 0.05 |
| **H30** | 0.70 | 0.70 | 0.70 | 0.70 | 0.00 |
| **Che** | 0.10 | 0.10 | 0.10 | 0.10 | 0.10 |
| **Aq** | balance | | | | |

| **Stability at 43°C (days)** | | | | | |
|---|---|---|---|---|---|
| | 211 | 211 | 211 | 211 | 53 |

The difference between Example 12 and Comparison B (which are otherwise identical) shows clearly that the addition of the hydrophobic silica noticeably increased the stability of the emulsion.

### Test for Formulation stability.

Each formulation was charged carefully into a sample bottle up its neck approximately 24 hours after its preparation, and the bottles transferred into a temperature controlled chamber in trays to minimise the risk of being in advertently tipped, shaken or knocked over. Each sample was removed from the chamber for assessment and returned carefully as soon as possible afterwards, always maintaining an upright orientation and avoiding shaking. The samples were visually assessed by an experienced observer at approximately weekly intervals (7 or 8 days apart) for the first 12 weeks of storage and thereafter either once or twice a month. The data given above is the last assessment when the sample passed.

The sample was considered to have failed the assessment when it satisfied one or more of the following criteria:-
**Phase separation/Coalescence -** thinning at the bottom of the bottle, droplets of oil at the top of the emulsion or a layer of oil phase at the top and/or a layer of water at the bottom
**Creaming -** the top of the emulsion looks thicker and creamier than the rest of the emulsion
**Cracks in the emulsion:** - which can be anywhere from the top of the bottle or down the sides and are usually a first sign that the emulsion is going to separate soon afterwards.
**"Pearlised" appearance: -** noticeable loss of formulation clarity..
   **Other significant visual change** - eg change of colour.

## Claims

1. An aqueous emulsion comprising
a continuous aqueous phase constituting at least 85% by weight of the emulsion and comprising a solution of an astringent antiperspirant salt,
a disperse oil phase constituting not greater than 10% by weight of the emulsion and comprising at least one water-immiscible oil, including fragrance oil
at least one aliphatic emulsifier at a concentration of at least 1.5% by weight and
a hydrophobic silica
in which
the antiperspirant salt is an aluminium-zirconium chlorohydrate optionally complexed with glycine and present at a concentration of at least 16%
the oil phase contains at least 0.7% by weight of fragrance oil
the emulsion satisfies a Fragrance Index of at least 15 and
the hydrophobic silica is present in an amount that generates a Stability Index of from 4 to 15.

2. A composition according to claim 1 in which the aqueous phase represents from 90 to 95% by weight of the emulsion.

3. A composition according to claim 1 or 2 in which the concentration of the aluminium salt is from 20 to 25% by weight of the emulsion.

4. A composition according to any preceding claim in which the emulsion satisfies a Fragrance Index of from 20 to 40.

5. A composition according to claim 4 in which the emulsion satisfies a Fragrance Index of from 22 to 33.

6. A composition according to any preceding claim which has a Stability Index of from 5 to 8.

7. A composition according to any preceding claim in which the oil phase comprises from 1 to 1.5% by weight of the fragrance oil blend, based on the emulsion.

8. A composition according to any preceding claim in which the fragrance oil blend represents from 10 to 50% by weight of the oil phase.

9. A composition according to claim 7 claim in which the fragrance oil blend represents from 20 to 40% by weight of the oil phase.

10. A composition according to any preceding claim in which the oil phase represents from 2 to 6% by weight of the emulsion.

11. A composition according to any preceding claim in which the oil phase comprises at least one triglyceride oil.

12. A composition according to claim 8 in which the triglyceride oil is sunflower seed oil.

13. A composition according to any preceding claim in which the emulsion contains from 2 to 5% by weight of the aliphatic emulsifier.

14. A composition according to any preceding claim in which the emulsion contains from 0.5 to 1.0% by weight of the hydrophobic silica.

15. A composition according to any preceding claim in which the antiperspirant active is aluminium zirconium tetrachlorhydrex gly at a concentration of above 20% by weight.

16. A composition according to any of claims 1 to 14 in which the antiperspirant active is aluminium zirconium pentachlorohydrate.

17. A method of stabilising an aqueous antiperspirant emulsion comprising
a continuous aqueous phase constituting at least 85% by weight of the emulsion and comprising a solution of an astringent antiperspirant salt,
a disperse oil phase constituting not greater than 10% by weight of the emulsion and comprising at least one water-immiscible oil, including fragrance oil
at least one aliphatic emulsifier at a concentration of at least 1.5% by weight and
a hydrophobic silica
**characterised in that** in said emulsion
the antiperspirant salt is an aluminium-zirconium chlorohydrate optionally complexed with glycine at a concentration of at least 16%,
the oil phase contains at least 0.7% by weight of fragrance oil and the emulsion satisfies a fragrance index of at least 15 %s being by weight based on the emulsion
by incorporating into the emulsion the hydrophobic silica in an amount that generates a Stability Index of from 4 to 15.

18. A method according to claim 17 which is **characterised by** any one or more of limitations a) to i)
a) the concentration of the aluminium salt is from 20 to 25% by weight of the emulsion
b) the aqueous phase represents from 90 to 95% by weight of the emulsion
c) the oil phase comprises from 1 to 2.5% by weight of the fragrance oil blend, based on the emulsion
d) the emulsion has a Fragrance Index of from 20 to 40
e) the emulsion has a Fragrance Index of from 22 to 33
f) the fragrance oil blend represents from 10 to 50% by weight of the oil phase
g) the fragrance oil blend represents from 20 to 40% by weight of the oil phase
h) the oil phase represents from 2 to 6% by weight of the emulsion.
i) which the aliphatic emulsifier comprises at least one triglyceride oil.
j) which the emulsion contains from 2 to 5% by weight of the aliphatic emulsifier
k) from 0.5 to 1.0% by weight of the hydrophobic silica is incorporated into the emulsion.
l) the emulsion has a Stability Index of from 5 to 8 and
m) the antiperspirant active is aluminium zirconium pentachlorate at a concentration of above 20% by weight.
